# EUROPEAN PATENT SPECIFICATION

(11) **EP 0 515 667 B1**
(45) Date of publication and mention of the grant of the patent: **28.02.1996**
(21) Application number: 92903101.1
(22) Date of filing: 16.12.1991
(51) Int. Cl.: A61N 1/30

(54) **HYDRATABLE IONTOPHORETIC BIOELECTRODE AND METHOD OF PREPARATION**
DURCH HYDRIEREN AKTIVIERBARE BIO-ELEKTRODE FÜR IONTOPHORESE UND VERFAHREN ZU IHRER HERSTELLUNG
BIOELECTRODE IONTOPHORETIQUE HYDRATABLE ET PROCEDE DE PREPARATION

(30) Priority: 14.12.1990 US 627714
(43) Date of publication of application: 02.12.1992
(73) Proprietor: IOMED, INC., Salt Lake City, Utah 84119-1449 (US)
(72) Inventor: LINDSAY, Lloyd, B., West Jordan, UT 84088 (US); BECK, Jon, E., Salt Lake City, UT 84108 (US); PETELENZ, Tomasz, E., Salt Lake City, UT 84108 (US); JACOBSEN, Stephen, C., Salt Lake City, UT 84102 (US)
(74) Representative: Belcher, Simon James
(86) International application number: PCT/US91/09329
(87) International publication number: WO 92/10235

(56) References cited:
- FR-A- 2 452 938
- US-A- 3 998 215
- US-A- 4 593 053
- US-A- 4 684 558
- US-A- 4 777 954
- US-A- 4 921 475
- US-A- 4 927 408
- US-A- 4 989 607
- US-A- 5 053 001

## Description

### BACKGROUND OF THE INVENTION

### Field of Invention

This invention concerns an iontophoretic bioelectrode incorporating a hydratable element and product. Particularly, this invention concerns an iontophoretic bioelectrode system for delivery of drugs, and two methods of preparing the iontophoretic bioelectrode and the hydratable product.

### Background Art and Related Disclosures

Iontophoretic bioelectrodes, used in place of hypodermic needles to inject medications into and through a person's skin, have typically used pouches or capsules, fibrous mats or sponges, or hydrogel monoliths to contain the medicament. In use, a typical medication solution containing ions is added to said capsule, or the pouch, the fibrous mat or sponge, or to the surface of the hydrogel depending on the electrode type.

With all these types of electrodes, the drug containing portion is interfaced with the skin and an electric current is passed through a suitable contact on the opposite side of the drug containing member, through the drug containing element and underlying skin, to the surrounding and underlying tissues. A second bioelectrode, placed on the skin in close proximity to the iontophoretic electrode, completes the electrical circuit. Positively charged ions in the medicament solution will migrate in response to this current flow in a direction away from the positive electrode and vice-versa for negative ions. If these ions are drug ions, proper choice of polarity and current level can result in their controlled introduction into and through the skin. Alternatively, uncharged drug molecules contained in a solution of non-medicament ions may also be delivered into and through the skin using these iontophoretic electrodes via the phenomenon of electroosmosis. Electroosmosis results from the flow of non-medicament ions in response to an electric current dragging a portion of the uncharged solvent in the same direction. The moving solvent, in turn, can carry with it a portion of dissolved uncharged drug molecules.

Iontophoretic bioelectrodes have been described earlier which incorporate pouches to contain aqueous drug solutions. This requires that the drug must penetrate the pouch wall in order to contact the skin. Previous electrode types using capsules for drug solution containment use a semipermeable membrane skin interface through which the drug must also penetrate. Alternatively, open-faced capsule electrodes with tacky, hydrophobic sealing rings which seal the capsule contents against the skin have been described. The first two electrode types suffer from a loss of drug delivery efficiency inherent with interposing an additional membrane barrier against the skin. Intimate skin constant is also relatively difficult to achieve as is conformability to odd skin geometries. The open faced electrodes have improved skin contact and drug delivery efficiency but suffer from a tendency to leak, particularly when applied to odd skin geometries. All these types of electrodes are relatively difficult to load as they must be loaded while attached to the patient, and all air bubbles must be removed from the drug capsules or pouches. Furthermore, an additional special device is required for filling these pouch or capsule electrodes such as filling valve or self-sealing septum.

Fibrous mat or sponge iontophoretic bioelectrodes have also been described. These electrodes incorporate woven or nonwoven mats of fabric or sponges to contain the aqueous drug solution. These electrodes suffer uniformly from poor drug solution containment and variable degrees of conformability and skin contact. Their tendency to leak drug solution results in air bubble inclusions and a consequent lack of a uniform electric current distribution in use. This can lead ultimately to electrical skin burns.

Considerations of the above difficulties have led to development of iontophoretic bioelectrodes incorporating hydrogels of various types for drug solution containment. Hydrogel members of the proper consistency will not leak drug solution, are very conformable, can be interfaced directly with the skin, provide intimate skin contact, and tend to hydrate the skin, thus improving iontophoretic drug delivery. These electrodes suffer, however, from several practical difficulties which limit their utility. Hydrogels, with their high water content, are subject to biological contamination and degradation. This requires addition of preservatives to the hydrogel and the consequent possibility of unwanted preservative-drug interactions. In addition, compatibility with metallic electrode components, skin fixation adhesives, and other components can be compromised by long-term exposure to this aqueous environment. Pre-drug-loaded hydrogel electrodes must be examined for degradation and drug compatibility of all components. An alternative method of loading a hydrogel bioelectrode at the time of use with drug requires at least several hours for the drug to slowly diffuse homogeneously throughout the body of the hydrogel. Hydrogel types which rapidly absorb aqueous solutions from a dry state have been intrinsically too ionic to serve as useful drug containing elements for this application because their inherent ions compete with those in the drug for transport into or through the skin.

US-4777957 discloses a bioelectrode for iontophoretic delivery of a medicament into the skin or tissue of a person or animal, which comprises a hydratable structure for absorbing ionised medicament when placed in contact therewith, an electrode for receiving an electrical current to produce an electrical field and cause ionised medicament to move from the hydratable element into the skin or tissue on which bioelectrode is placed, the electrode being mounted on the holding means in proximity to the hydratable element, and means for holding the hydratable element and the electrode together.

Disadvantages outlined above are obviated by the new hydratable hydrogel product of this invention which is made an integral part of such iontophoretic bioelectrode. The resulting bioelectrode is simple, inexpensive, conformable to the skin contour and includes an element of hydratable material which rapidly absorbs and retains aqueous solution preferably containing medicaments for iontophoretic delivery.

### SUMMARY

One aspect of the invention is a simple, inexpensive, skin contour conformable iontophoretic bioelectrode incorporating a new hydratable element product, and a method for preparation of said bioelectrode and hydratable product.

Another aspect of the invention is an iontophoretic bioelectrode capable of efficiently absorbing and retaining an aqueous solution in its rehydratable structure when placed in contact therewith.

Still another aspect of the invention is an iontophoretic bioelectrode incorporating a dry hydratable product for convenient loading which rapidly absorbs and retains medicament which when hydrated behaves essentially as a monolithic gel element.

Still another aspect of the invention is an iontophoretic bioelectrode for iontophoretic delivery of the drug, further incorporating means of fixation to the body, and a electrically conductive element.

Still yet another aspect of the invention is an iontophoretic electrode wherein the drug is applied to and contained in a hydratable product area where the need for additional devices allowing filling the hydratable element, or for sealing ring to prevent leakage of the drug liquid, is obviated.

Still yet another aspect of the invention is to provide the bioelectrode which is stable and storable in a dry form until used, thereby reducing materials incompatibility problems, and contamination problem. Because drug is added at time of use, there is no problem with drug storing.

Still yet another aspect of the invention is the hydratable element product comprising a stack of a plurality of hydrogel sheets for absorbing medicament said hydratable structure further comprising means for maintaining adjacent sheets at least partially separated and means for holding the sheets in the stack.

### BRIEF DESCRIPTION OF DRAWINGS

Figure 1 is a perspective exploded view of individual components of the hydratable bioelectrode.

Figure 2 depicts the hydratable structure comprising a stack of gel layers separated by spacing means.

Figure 3 is a cross-sectional view of a raw material with scrim used in the preparation of gel layers of making the hydratable structure.

Figure 4 depicts peeling-off gel layer from the raw material containing scrim.

Figure 5 is a cross-sectional view of the roller device used for peeling-off the gel into two thinner layers.

Figure 6 depicts a process of swelling of gel layers.

Figure 7 illustrates how the electrode is drug loaded and attached to a patient's skin.

### DETAILED DESCRIPTION OF THE INVENTION

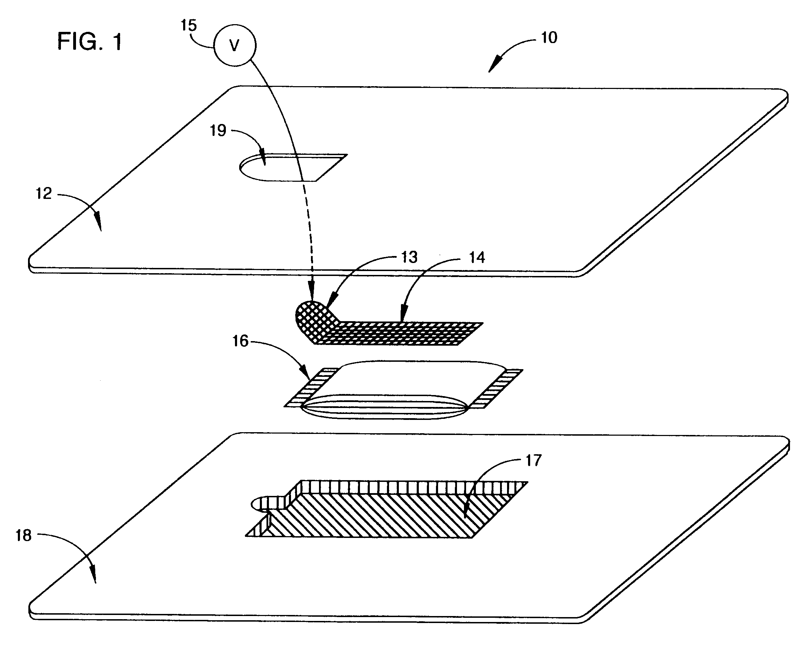

Current invention describes a new hydratable structure incorporated into an iontophoretic bioelectrode for delivering medicament into the skin, as well as the bioelectrode and a method of manufacture for such bioelectrode itself and hydratable structure. The hydratable bioelectrode dismantled into its components is shown in Figure 1.

The bioelectrode 10 is composed of an assembly of several different components which include a hydratable element 16 for absorbing and containing aqueous solutions of medicament when placed in contact therewith, a containment tray apparatus 18 for holding the hydratable element 16 prior to and during hydration, an electrical contact element 14 attached to the top of the hydratable element 16, and a tacky, adhesive, skin fixation sheet 12 overlying the hydratable element 16 and electrical contact 14. Sheet 12 is perforated with an access hole 19 for filling of the hydratable element with medicament solution and also for electrical connection of the electrical contact 14 with electrical source 15.

Finished and assembled dry bioelectrode 10 is a sandwich-like structure having on one side the adhesive coated film 12 and on the other side the containment tray 18. The medicament receptacle portion 17 of tray 18 is a formed sunken space into which both the hydratable element 16 and electrical contact element (electrode) 14 fit. The bended tip portion 13 of the conductor element 14 is connected with the electrical source 15 through the access hole 19 which also provides a port for pouring an aqueous solution of the drug onto and around the hydratable element.

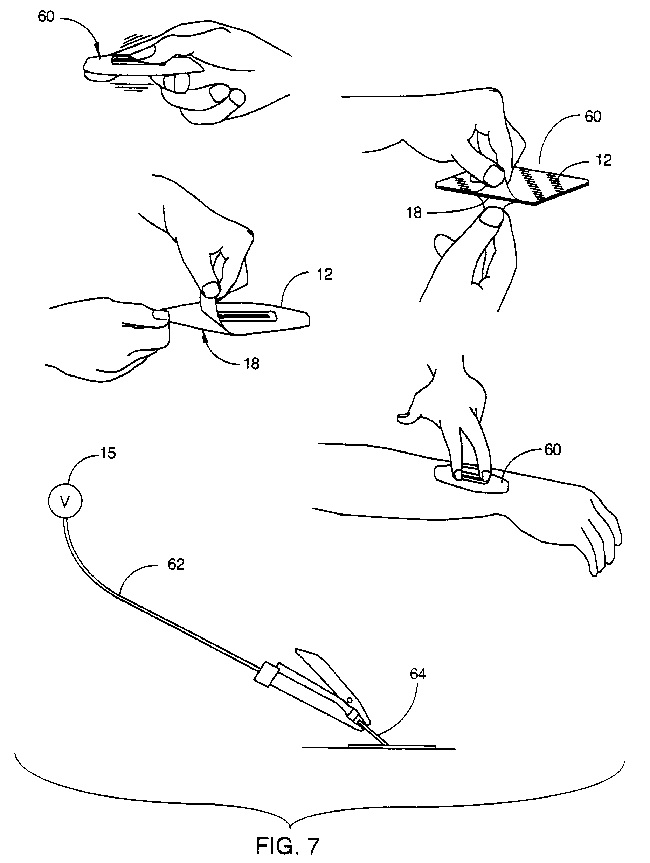

The adhesive film 12, electrical contact element 14, and hydratable element 16 are fastened together using flexible plastic (polyurethane) staples. The bioelectrode ready to use and its actual use is shown in Figure 7.

When the bioelectrode 10 is used for drug delivery to a patient, the receptacle 17 of the tray 18 is filled with the solution of drug which is absorbed into hydratable element 17. The tray 18 is then removed by peeling it off and then the hydrated element 16, conductive sheet 14 and adhesive coated film 12, stapled together are placed as a unit on the patient's skin with hydratable element 16 being in contact with the skin, and the adhesive film holds it in place.

The containment tray is made of styrene plastic sheeting which is surface coated with a silicon release agent. This material is commercially available from Mead Release Products, Dayton, Ohio.

The hydratable element 16 is initially dry, which eliminates the problem of the growth of biological contaminants and the need for added preservatives. The drug is added to the hydratable element only at time of use which further eliminates long term stability problems due to prolonged wet exposure to the electrode components.

In use, the hydratable element 16 performs dual functions in this design. It provides drug containment and also provides safe spacing between the skin and the metallic electrical contact 14. The containment tray 18 serves three purposes. During storage and prior to use, it provides protection as a packaging around the hydratable structure element 16. During the filling process when liquid medicament is introduced into the hydratable element 16, the containment tray 18 functions as a soaking receptacle inside which the hydratable element 16 is held in contact with the drug fluid. Finally, it serves as a release liner for the adhesive coated skin fixation film 12.

When connected to an iontophoretic electrical current source 15, the electrical contact element 14 in the bioelectrode distributes current over the upper surface of the hydratable element 16. In order to attach the bioelectrode to the skin surface, a method of fixation is provided. An adhesive coated film sheet 12 overlays the contact and hydratable elements adhering the bioelectrode to the skin.

The hydratable element 16 of this invention performs the functions listed above and also satisfies technical requirements of iontophoretic acceptability and practicality. Iontophoretic acceptability requires that the raw gel material itself be free of ions which would otherwise compete with the drug ions, be nontoxic and nonirritating, be stable under the conditions of storage and use, contain a large weight percent of water and allow relatively free mobility of the drug ions within the gel matrix. These criteria are satisfied through the proper choice of gelling polymer and degree of crosslinking of this polymer.

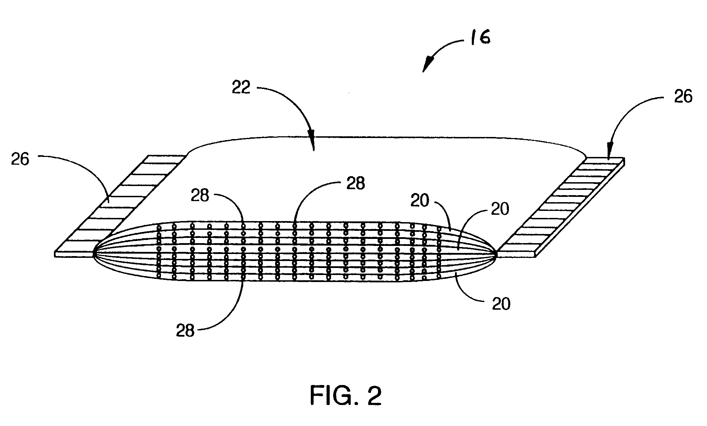

The hydratable element 16 in greater detail is seen in Figure 2. The hydratable element 16 is, constructed of plurality of sheets 20 of hydratable material forming a stack of the sheets (layers). The surface layers 22 and interior layers 20 are of the same material. The edges of the hydratable layers 20 and 22 are compressed into seams 26 which are pressed end portions of the convergent layers. Individual layers 20 are held slightly apart from each other by a spacing means 28.

A number of cross-linked high molecular weight non-ionic but hydrophilic natural and synthetic polymers are useful for manufacturing the hydratable element 16. These polymers include modified starches and cellulose, polyethylene oxide (PEO), polyacrylamide, polyvinyl alcohol, and copolymer mixtures of these polymers. Other suitable non-cross-linked polymers include block co-polymers of the hydrophilic polymers listed above with short sections of hydrophobic polymers. This latter group of polymers includes PEO/polyurethane block polymers which can swell with water but do not dissolve at room temperature because of hydrophobic knots composed of entangled polyurethane polymer sections. The polymer of preference is an electron-beam cross-linked PEO available commercially as a wound dressing.

The primary practical requirement for the polymer based element is that thorough and homogeneous hydration of the hydratable element must occur within a practical, short time, preferably in about one minute. This can only be achieved with a hydrogel if the surface area to volume ratio of the dry hydrogel polymer is kept high. In practice, this means that rapid hydration requires the dry gel be composed of a thin layer, a thin strand, or of small particle. For practical reasons, thin layers between 0.025 and 0.00025 cm (0.010 and .0001 inch) are preferred, with 0.0013 cm (0.0005 inches) being optimal in this case. Such sheet by itself, however, is too thin to provide for safe distance of the skin from the electrical contact to protect against burning or skin irritation. This invention solves the problem by simply providing a stack of an adequate number of 1 to 100 thin layers, preferably 25-35, into a hydratable structure element.

A further problem is connected with stacking the layers. When a stack of nonionic hydrophilic polymer layers is introduced to aqueous solution, their edges tend to immediately collapse together around a dry core in a manner similar to the clumping of corn starch when added to cold water. This phenomenon is known as "gel blocking" and normally prevents complete hydration of the hydratable element in this form. This problem is eliminated in this invention by adding two new features, namely stiffening and spacing.

Stiffening the dry gel layers is achieved with a suitable rapidly wetting, non-ionic, nontoxic, non-irritating excipient dried into the gel layer. The stiffening agent may be sucrose, glucose, sorbitol, or any other rapidly water soluble mono or disaccharide, preferably sucrose. Because the stiffening agent is dissolved upon hydration, conformability of hydrated element is unaffected. The spacing granules or fibers any also be composed of non-dissolving compounds such as cellulose fibers. Spacing the layers apart with granular or fibrous spacing elements 28 prevents gel blocking. The spacing means placed in between two adjacent layers 20 needs be no larger than the thickness of the layers in order to achieve a capillary effect which allows wicking of the drug solution to the core of the electrode. Spacing is typically achieved by depositing granules in a random pattern, fibers or other structures between two adjacent sheets.

Hydration of such an element requires that the drug solution wicks to the entire surface of each layer before the stiffening agent and spacing granules can dissolve. This last criteria practically limits the width of the layers to about one inch for the size granules need here. As the element hydrates, the spacers in the case of sugar dissolve and the fully hydrated layers swell together to form what is essentially a monolithic hydrogel slab with homogeneous drug content throughout. When cellulose is used as a spacer, the cellulose fibers are surrounded and incorporated in the gel mass which also takes the form of a gel monolith.

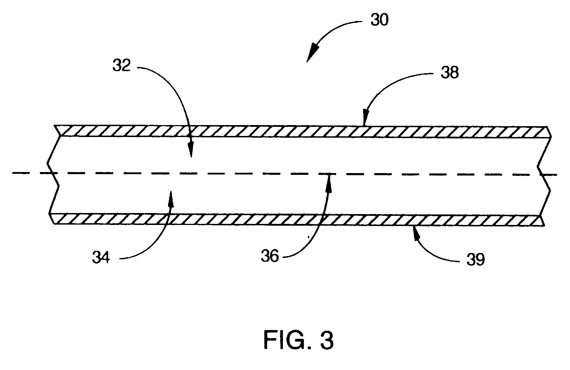

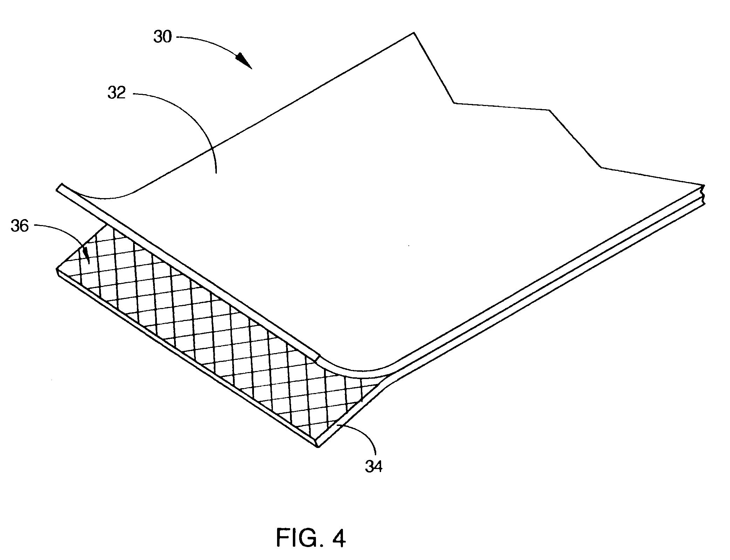

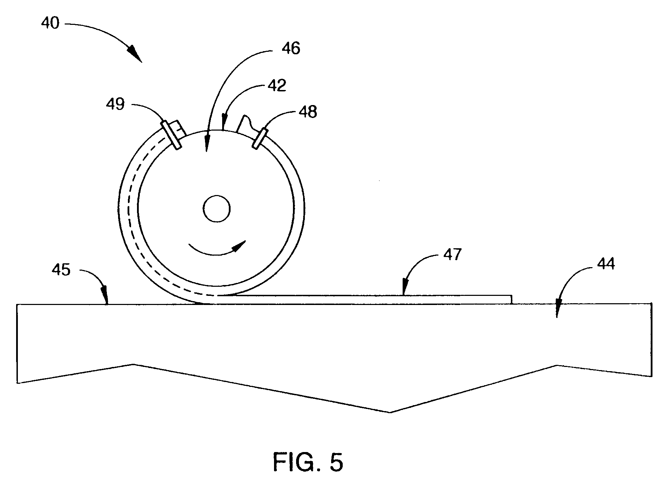

The gel raw material, seen in Figures 3 and 4, is composed of a sheet 30 of radiation crosslinked high molecular weight PEO available commercially as a wound dressing. The gel sheet is enclosed with removable polyethylene liners on either side and contains a thin polyethylene reinforcing scrim which bisects the gel layer, as seen in Figures 3 and 4. Processing requires that the scrim 36 be removed. This separates the gel above the scrim 32 from the gel below the scrim 34. Thus, for every piece of scrimmed raw material descrimmed, two, half-thickness pieces of gel result. The descrimming process is illustrated in Figures 4 and 5. (In Figure 4, the liners 38 and 39 are not shown). Alternatively, the gel is available in a sheet half as thick enclosed between two removable liners 0.0076 and 0.0025 cm (.003 inches thick and 0.001 inches) thick but without the scrim.

The manufacturing process for construction of the hydratable element resolves itself into several unique steps.

The first process step involves removing and discarding one liner 38 (Figure 3) from the surface of gel raw material 30. With the scrimmed material, this is easily accomplished by hand. For the non scrimmed material, this is more difficult requiring handling fixtures of special design and construction which hold the thicker liner with vacuum while stripping the other liner with a peeling fixture. Alternatively, the latter material may be frozen and the thin liner peeled by hand.

The scrim is then removed from the raw material, for example, by splitting the gel sheet roughly in half at the scrim. This results in two pieces of unscrimmed gel of comparable thickness. Specially designed splitting fixtures are shown in Figure 5.

Figure 5 shows the peeling fixture 40 and illustrates the peeling process. The fixture 40 comprises a roller 46, two clamps 48 and 49 for holding a layer of the gel and a cold, freezing table 44. One sheet of the unfrozen raw gel material 30, with liners removed is attached with clamps 48 and 49 to a surface 42 of the roller. The roller is then rolled along the freezing surface 45 of the table 44 and the frozen layer of gel 47 is peeled away from the roller 46 and remains on the table.

Splitting process involves rolling one side of the intact gel sheet onto a cold surface allowing the layer of gel in contact with the surface to freeze partially through the thickness of the layer and then pulling the unfrozen layer of gel away from the surface. This action causes the gel to separate at the scrim which then lifts off with the unfrozen gel layer. Since the scrim now rides on the surface of this remaining layer, it may then simply be lifted off the unfrozen gel section and discarded. The unscrimmed material, of course, does not require this treatment. In this way, individual hydrogel layers of the hydrogel element are prepared by further submitting them to the rest of the process steps.

The raw material sections, whether formerly scrimmed or not, are then dried or thawed (while attached to teflon trays or polyethylene liner) at temperature 40-70°C, preferably at 60°C, in a drying chamber of local design and construction.

Gel sheets dried in this manner have the unique property, when rehydrated with aqueous solution, of expanding laterally to more than twice their original lateral dimensions. In doing so, their thickness dimension decreases from .03 inches (hydrated) to less than .01 inches. This thickness decrease aids in achieving the conditions for rapid hydration mentioned above. If a sugar solution of between 1 and 10% in deionized water, preferably 2-4%, is used to rehydrate and swell the membranes, an adequate degree of stiffness is achieved in the dried swollen membranes.

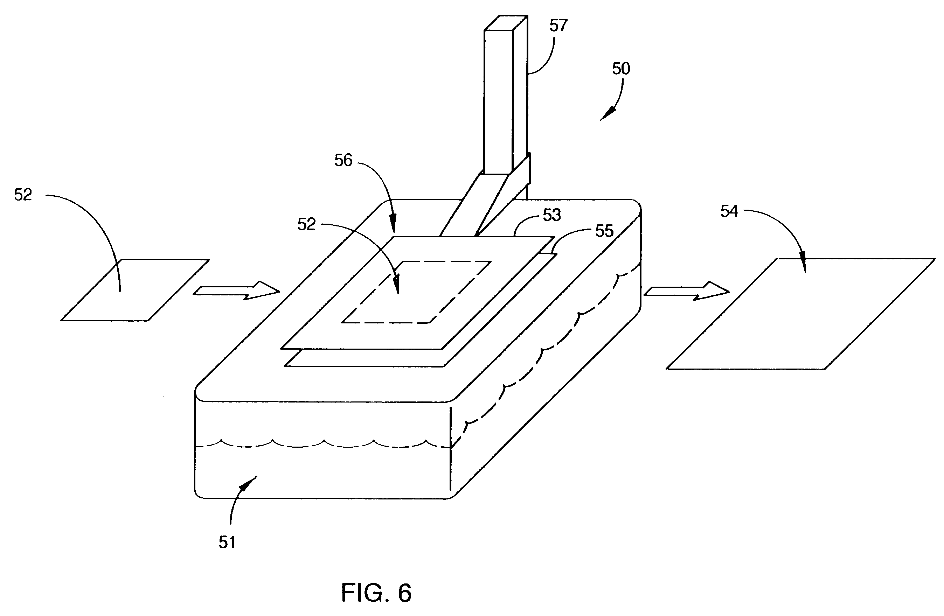

The reswelling and expanding of the membranes is illustrated in Figure 6. Figure 6 show schematically an apparatus 50 and a process for swelling of dried gel sheets. Dried gel sheet 52 having size approximately 15.24 x 15.24 cm (6 x 6 inches), is placed between two parallel screens 53 and 55 mounted on the holder 57 attached to the swelling apparatus 50. The basket 56 is vertically movable along the holder 57 so that it can be completely or partially submerged in the swelling tank 51. Typically, the swelling is accomplished by placing a piece of the dried material in between the screens 53 and 55 which are positioned horizontally in the bath of sugar solution. The center point of the gel piece 52 is pinned in a place while the screens 53 and 55 which are attached to a programmable linear drive fixture slowly oscillated vertically beneath the surface of the solution. This process takes 1-2 minutes. The basket 56 is then removed from the bath 51, excess fluid removed, and the expanded thin gel sheet redried, again at 60°C.

The dried expanded gel sheets, on trays, are then placed on a conveyor which runs under a commercial sugar deposition machine set to deposit, usually by dropping an appropriate density of sugar particles, (1.8 gram sugar on a 27.94 x 43.18 cm (11" x 17") membrane area). The sugared membranes are then misted with deionized water to partially melt the sugar particles and cause them to adhere to the membrane. The membranes are then again dried to less than 1% residual moisture and peeled from the trays.

The finished membranes are stacked as described above. These stacks are then compressed in several narrow parallel streaks which forms the end seams on the finished elements and cut along the seams into strips. The distance between the seams determines the finished element length. These strips are then run edgewise into a commercially available slicing machine with blades set to positions which determine the width of the element. The cutters on this machine oscillate with reciprocating motion which prevents the element edges from shearing together and sealing-off the hydration spaces between the gel layers.

In practice of cutting the rehydratable structure, the one large stack is first cut and crimped long its longest edges, and then cut perpendicularly to the press-cut forming thus providing a plurality of individual stacks of gel layers, each of which may then be incorporated into a bioelectrode structure such as that shown in Figure 7.

When the ionized medication is introduced into the hydrogel layer, it holds and retains it until the electrical current is applied to the electrode which causes the transport of the medicament through the skin.

The final dry bioelectrode 60 is constructed from an adhesive coated film, an electrical contact element, a hydratable element and a containment tray. The dry bioelectrode looks like a pad mounted on a plastic tray. The hydratable element is placed in a recessed cavity in the tray. The electrical contact element is placed above it, and both hydratable element and electrical contact are covered with and stapled to the adhesive coated film. The individual elements are seen in Figure 1. The assembled electrode prepared for use is seen in Figure 7.

The actual use of the bioelectrode is described in Example 3. Typically, prior use, the drug such as, for example, dexamethasone, lidocaine, epinephrine, to name a few, is dissolved in an aqueous solvent and added to the receptacle 17 holding the hydratable element 16. The surface of the electrode is gently pressed along side to assure the even distribution of the drug in the hydratable element. The surface of the electrode is gently pressed alongside to assure the even distribution of the drug in the hydratable element. A short period of time is allowed for hydration. The plastic containment tray is then removed and the bioelectrode is placed on patient's skin. The hydratable element in contact with the skin and hydrated with the drug adhesive film, which is larger than hydratable element, is gently attached to the skin. There is no contact between the skin and the electrical contact element. The contact element, which is above the electrical hydratable element is connected with the electrical source, such as for example, Phoresor unit, available from IOMED Corp. and 0.1-4 mA current is applied for several minutes, depending on the drug, treatment and the patient.

### UTILITY

The iontophoretic bioelectrode in which the ionized medicament is absorbed has a plurality of hydratable gel layers which are structured to comprise the hydratable element. Each hydratable layer is separated from adjacent layers by granules or fibers. This separation provides spacing between successive layers which serve as pathways, or flow channels. When introduced to an aqueous solution of medicament, the liquid is then distributed over the entire surface of each of the gel layers. Each layer swells as it absorbs and retains the liquid, eliminating the interlayer spacings and incorporating the spacing granules (which subsequently dissolve in the case of sucrose or other mono or disaccharides) or fiber into the gel. This structure essentially becomes a gel monolith which completely contains the drug solution. When an electrical current is applied to the electrical contact, which is in contact with one side of the gel, ions in the gel matrix migrate toward and into the skin. The high water content, the uniform distribution of ionic drug solution, and the soft viscoelastic properties of the gel material provide for improved conformability and skin contact as well as enhanced tissue wetting. This improves iontophoretic bioelectrode performance.

These and other embodiments of the current invention are intended to fall within the scope of the invention.

### EXAMPLE 1

### Preparation of Rehydratable Product Element for the Iontophoretic Bioelectrode

This example illustrates a preparation of the hydratable structure component for the iontophoretic electrode of this invention.

The process of preparation of the hydratable element begins with cutting a strip of approximately 6 inches by 13 inches from the polyethylene oxide (PEO) stack material in conventional fashion using the standard cutting table with mounted cutting fixture. The 15.24 x 33.02 cm (6 x 13 inches) strip of PEO was placed on a flat table top and 5.08 x 7.62 cm (2 to 3 inches) of the linear were peeled off the gel along one of the shorted edges of the strip. The short edge of the strip having peeled the liner was fastened into the peel roller clamp with the exposed gel facing outwardly. The rest of the remaining liner was removed from the outfacing side of PEO and this free edge was fastened with the second clamp. A fluoro-coated tray was positioned onto the cold table, to cool the tray and vacuum was applied in conventional manner to hold the tray in place. The tray was allowed to reach a steady state temperature of about -7.8°C (18°F) (a temperature below the freezing point of the gel layer).

The peeling roller with gel layer wound around it was aligned along fluoro-tray edge and the entire length of the layer of frozen PEO was peeled off. Basically, the roller, with gel layer wound thereabout, was aligned along one edge of the cooled tray and rolled at a predetermined, controlled rate to cause the outward facing or upper layer of the gel material to freeze and hold onto the tray so that, as the roller continues to roll, the thin layer was peeled away from the remainder of the gel on the roller and frozen onto the tray. If no scrim was present in the gel mass, the tray temperature and rate of rolling the roller determined the thickness of the layer of gel 38 which was frozen to the tray and peeled from the roller. If the layer of the scrim mesh material 36 was positioned in between two layers of the PEO, the position of the scrim determined the thickness of the PEO layer.

The vacuum was then released, the tray was removed from the cold table and placed on shelf of the transportation rack. With the layer of gel on the tray, the tray was placed in a convection drying chamber which has been heated to about 55°C. The purpose of this was to dry the gel layer at a temperature which will not cause degradation of the gel, typically about 60°C. The sheets were dried for approximately 90 minutes or until they were fully dried.

Trays with dried PEO were removed from drier chamber using a roller rack to transport them. Dried PEO were peeled from the fluoro-coated trays. If there was an excess of PEO layers, these were stored in a desiccator cabinet.

The dried gel layer which was removed from the tray was placed onto a screen and clamped to maintain the planarity of the layer and the screen was then submerged into a swelling bath fixture containing a swelling solution of water, additionally containing a sugar as a stiffening agent in amount of 50 grams per liter for about 2 minutes. The screen on which the gel layer was placed was a perforated fluoro-coated metal sheet. Another screen was placed on top of the gel layer to maintain the flatness of the gel layer.

The screen with gel layer remained submerged in the swelling solution for a sufficient time to allow the layer to absorb solution, swell and expand laterally. The screen with swollen gel layer was then removed from the swelling solution and dried, being first set-up on a drip tank for approximately 1 minute. The screens with drip-dried PEO were placed on transportation rack, rolled and loaded into a stage II-drier, a convection drying chamber was used to further dry the gel layer for approximately 60 minutes at 60°C. After swelling and the final step of drying, the resulting gel layer had substantially the same length and width dimensions, but the thickness of the layer had decreased substantially from when wet.

The dried, swollen PEO was placed onto a transportation rack and moved to a granular dispersing unit.

In the next stage of the process, granules or fibers were distributed onto the gel layer to serve as spacers to maintain apart, to the extent possible, adjacent gel layers which will later be used to form a stack of gel layers.

Dried, swollen PEO, still on the screens, were transferred onto the conveyor belt of the granulation dispenser, one at a time, and each tray was passed under the dispenser and sucrose particles were dropped on the dried PEO sheets, using 1.8 g of sucrose on 27.94 x 43.18 cm (11 x 17 inches) area.

The screens with PEO and granules dispersed thereon were then transported to the misting station where the PEO and granules were dampened via misting. A fine water vapor or mist was applied to the gel layer simply to better hold the granules or fibers on the gel layer surface. The water vapor or mist partially dissolved sugar granules, causing them to stick onto the gel layer. It is important that the amount of water vapor or mist used is such that the granules are not dissolved completely, because that would defeat the main purpose of having the granules there, namely to serve as spacers to maintain the gel layer separated from adjacent layers.

The screens with damped PEO with granules were moved again to the stage II drier and dried again at about 55°C for about 20 minutes.

After securing the granules or fibers onto the gel layer, the gel layer was removed from the screen and then arranged in a stack with other gel layers, for a total of 28 layers of dried/granuled PEO sheets. In other cases, the number of layers was different. However, sufficient number of gel layers was always included in a stack so that when the gel layers were incorporated into a bioelectrode such as that shown in Figures 1 and 7, the electrode 14 which receives electrical current from a current source 15 is sufficiently removed from and does not burn the skin or tissue of a person to be treated.

The dried PEO-granule sheets were arranged in sheet stacks using the stack aligning fixture. The stack was delivered to the roller cutter, loaded onto the press cut fixture, and was press-cut by a roller press which both cuts the stack lengthwise, and also crimps the resulting adjacent edges so cut into the hydratable element of the iontophoretic electrode.

### EXAMPLE 2

### Assembling of an Iontophoretic Bioelectrode

This example illustrates assembling of the dry iontophoretic bioelectrode.

The hydratable product obtained in Example 1 was then mounted together with the electrode sheet onto a containment tray, as seen in Figure 1, and covered with adhesive coated film. In this dry form, the bioelectrode was stored until used.

### EXAMPLE 3

### Hydration of the Bioelectrode and the Use for the Drug Delivery

This example illustrates the hydration of the hydratable element with the drug solution and use of the bioelectrode for drug delivery to a patient. The procedure is schematically shown in Figure 7.

The drug in solution was added to the medicament receptacle portion of tray where the hydratable element (in dry form) is placed as in Example 2. The hydrated bioelectrode 60 was firmly squeezed to distribute the medication along its full length and 1-2 minutes were allowed for uniform hydration of the hydratable element.

Skin site where the bioelectrode is to be applied was cleaned with alcohol. After the area is completely dry, the bioelectrode containing the hydratable product and chemical contact element attached by staples to the adhesive coated film 12, was peeled from the containment tray 18, and applied to the patient's skin. There is no need to tape, compress or bid the bioelectrode. Adhesive film 12 keeps the bioelectrode in place.

Connecting leads 62 from the electrical source unit 15 were connected to a tab 64 protruding through the access hole from the electrode 2.0 mA current was applied for 20 minutes.

## Claims

1. A bioelectrode (10) for iontophoretic delivery of a medicament into the skin or tissue of a person or animal, which comprises:
(a) a hydratable structure (16) for absorbing ionised medicament when placed in contact therewith,
(b) an electrode (14) for receiving an electrical current to produce an electrical field and cause ionised medicament to move from the hydratable element into the skin or tissue on which bioelectrode is placed, the electrode being mounted on the holding means in proximity to the hydratable element, and
(c) means (12) for holding the hydratable element and the electrode together,
characterised in that the hydratable element comprises a stack of at least two sheets (20) of hydrogel for absorbing medicament, the hydratable structure including means (28) for maintaining adjacent sheets at least partially separated, and means (26) for holding the sheets together in the stack.

2. A bioelectrode as claimed in claim 1, in which the hydrogel sheets (20) comprise a composition of a polymer and sugar.

3. A bioelectrode as claimed in claim 2, in which the polymer is polyethylene oxide, polyacrylamide, polyvinyl alcohol, modified starch, cellulose, or co-polymers of these polymers.

4. A bioelectrode as claimed in any one of claims 1 to 3, in which the separating means (28) are granules or fibres disposed between each pair of adjacent hydrogel sheets (20) to allow the flow of medicament between the sheets.

5. A bioelectrode as claimed in claim 4, in which the separating means (28) comprise granules in the form of sugar crystals or cellulose particles.

6. A bioelectrode as claimed in any one of claims 1 to 5, in which the hydrogel sheets (20) are held together by one of crimping, pressing and cutting the opposite edges (26) of the sheets.

7. A bioelectrode as claimed in any one of claims 1 to 6, which includes a containment tray (18) having a medicament receptacle.

8. A bioelectrode as claimed in any one of claims 1 to 7, in which the means (12) for holding the hydratable element and the electrode together is an adhesive film.

9. A method of making a bioelectrode for iontophoretic delivery of a medicament, which comprises:
(a) forming the mass of hydrogel into sheets (20),
(b) drying the sheets of hydrogels,
(c) disposing the dried sheets in a swelling solution to cause the sheets to absorb solution, swell and expand laterally,
(d) drying the sheets of swollen hydrogel,
(e) distributing granules (28) or fibres onto the sheets,
(f) arranging the sheets into a stack such that they are at least partially maintained apart by the granules or fibres,
(g) applying to the stack of sheets an electrode (14) for receiving an electrical current to produce an electrical field and cause ionised medicament to move from the hydratable element into the skin or tissue on which bioelectrode is placed, the electrode being mounted on the holding means in proximity to the hydratable element.

10. A method as claimed in claim 9, in which step (a) comprises:
• placing the mass of hydrogel on the exterior on the exterior surface of a roller (46),
• rolling the roller with hydrogel over the surface of a freezing tray (45, 44) so as to cool an outer layer of gel as it contacts the table, causing it to freeze superficially, adhere to the tray, and
• pulling the tray away from the hydrogel mass remaining on the roller, to form a sheet of hydrogel.

11. A method as claimed in claim 9 or claim 10, in which step (b) comprises drying the sheets of hydrogel in a convection oven at a temperature between 45 and 65°C.

12. A method as claimed in any one of claims 9 to 11, in which step (c) comprises:
• placing the sheets of hydrogel on a screen,
• immersing the screen with hydrogel sheets in a solution of water and stiffening agent to cause the sheets to swell and ultimately to stiffen.

13. A method as claimed in any one of claims 9 to 12, in which step (e) comprises:
• moving the sheets of hydrogel under a granule or fibre dispensing mechanism to deposit the granules or fibres to facilitate holding them in place on the sheets, and
• applying water mist or vapour to the sheets and granules or fibres to moisten the granules or fibres to facilitate holding them in place on the sheets.

## Patentansprüche

1. Bioelektrode (10) für die iontophoretische Abgabe eines Medikaments an die Haut oder das Gewebe einer Person oder eines Tieres, welche umfaßt:
(a) eine hydratisierbare Struktur (16) zur Absorption eines ionisierten Medikaments, wenn dieses in Kontakt damit gebracht wird
(b) eine Elektrode (14) zur Aufnahme eines elektrischen Stroms, um ein elektrisches Feld herzustellen und das ionisierte Medikament zur Wanderung von dem hydratisierbaren Element in die Haut oder das Gewebe, auf welchem die Bioelektrode plaziert ist, zu veranlassen, wobei die Elektrode in der Nähe des hydratisierbaren Elements auf der Haltevorrichtung angebracht ist und
(c) Vorrichtungen (12) zum Zusammenhalten des hydratisierbaren Elements und der Elektrode,
dadurch gekennzeichnet, daß das hydratisierbare Element einen Stapel von mindestens zwei Schichten (20) von Hydrogel zur Absorption des Medikaments umfaßt, wobei die hydratisierbare Struktur Mittel (28) einschließt, um benachbarte Schichten zumindest teilweise getrennt zu halten, und Mittel (26), um die Schichten zusammen in dem Stapel zu halten.

2. Bioelektrode nach Anspruch 1, bei der die Hydrogelschichten (20) eine Zusammensetzung aus einem Polymer und Zucker enthalten.

3. Biolektrode nach Anspruch 2, bei der das Polymer Polyethylenoxid, Polyacrylamid, Polyvinylalkohol, modifizierte Stärke, Cellulose oder Copolymere dieser Polymeren ist.

4. Bioelektrode nach einem der Ansprüche 1-3, bei der die Trennvorrichtungen (28) Körner oder Fasern sind, die sich zwischen jedem Paar benachbarter Schichten (20) befinden, um den Medikamentenfluß zwischen den Schichten zu erlauben.

5. Bioelektrode nach Anspruch 4, in der die Trennmittel (28) Körner in der Form von Zuckerkristallen oder Cellulosepartikeln umfassen.

6. Bioelektrode nach einem der Ansprüche 1-5, in der die Hydrogelschichten (20) zusammengehalten werden durch Kräuseln, Verpressen, oder Schneiden der entgegengesetzten Kanten (26) der Schichten.

7. Bioelektrode nach einem der Ansprüche 1-6, die einen Behälterboden (18) einschließt mit einem Medikamentenbehältnis.

8. Bioelektrode nach einem der Ansprüche 1-7, bei der die Vorrichtung (12) zum Zusammenhalten des hydratisierbaren Elements und der Elektrode ein Klebefilm ist.

9. Verfahren zur Herstellung einer Bioelektrode zur iontophoretischen Abgabe eines Medikaments, welches umfaßt:
(a) Formen der Hydrogelmasse in Schichten (20),
(b) Trocknen der Hydrogelschichten,
(c) Einbringen der trockenen Schichten in eine Quellösung, um eine Absorption der Lösung durch die Schichten, ein Quellen und seitliches Expandieren zu erreichen,
(d) Trocknen der gequollenen Hydrogelschichten,
(e) Verteilung von Körnern (28) oder Fasern auf den Schichten,
(f) Anordnung der Schichten in einem Stapel in der Weise, daß sie zumindest teilweise durch die Körner oder Fasern getrennt voneinander gehalten werden,
(g) Anbringen einer Elektrode (14) an den Schichtstapel zur Aufnahme eines elektrischen Stroms, um ein elektrisches Feld zu erzeugen und das ionisierte Medikament zur Wanderung von dem hydratisierbaren Element in die Haut oder das Gewebe, auf welchem die Bioelektrode angebracht ist, zu veranlassen, wobei die Elektrode auf der Haltevorrichtung in der Nähe des hydratisierbaren Elements angebracht ist.

10. Verfahren nach Anspruch 9, bei welchem der Schritt (a) umfaßt:
- Plazieren der Hydrogelmasse auf dem Außenbereich auf der äußeren Oberfläche einer Walze (46),
- Rollen der Walze mit Hydrogel über die Oberfläche eines Gefrierbodens (45, 44) in der Weise, daß eine äußere Gelschicht gekühlt wird, wenn sie den Tisch berührt, wodurch sie oberflächlich gefriert und an dem Boden klebt, und
- Abziehen des Bodens von der Hydrogelmasse, die auf der Walze verbleibt, wobei eine Hydrogelschicht gebildet wird.

11. Verfahren nach Anspruch 9 oder 10, bei dem Schritt (b) das Trocknen der Hydrogelschichten in einem Konvektionsofen bei einer Temperatur zwischen 45 und 65 °C umfaßt.

12. Verfahren nach einem der Ansprüche 9-11, bei dem der Schritt (c) umfaßt:
- Plazieren der Hydrogelschichten auf einem Sieb,
- Eintauchen des Siebes mit Hydrogelschichten in eine Lösung von Wasser und Versteifungsmittel, wodurch die Schichten zum Quellen und schließlich zur Versteifung gebracht werden.

13. Verfahren nach einem der Ansprüche 9-12, bei dem der Schritt (e) umfaßt:
- Bewegen der Hydrogelschichten unter einer Vorrichtung zum Abgeben und Ablagern von Körnern oder Fasern, um das Aufder-Stelle-Halten der Schichten zu erleichtern, und
- Anwendung von Wassernebel oder -dampf auf die Schichten und Körner oder Fasern, um die Körner oder Fasern zum besseren Halt auf den Schichten zu befeuchten.

## Revendications

1. Bioélectrode (10) destinée à l'administration ionophorétique d'un médicament dans la peau ou le tissu d'une personne ou d'un animal, qui comprend :
(a) une structure hydratable (16) destinée à absorber un médicament ionisé lorsqu'elle est placée en contact avec ce dernier,
(b) une électrode (14) destinée à recevoir un courant électrique afin de produire un champ électrique et amener le médicament ionisé à migrer depuis l'élément hydratable jusque dans la peau ou le tissu sur lequel l'électrode est placée, l'électrode étant montée sur le moyen de fixation à proximité de l'élément hydratable, et
(c) un moyen (12) pour maintenir l'élément hydratable et l'électrode ensemble,
caractérisée en ce que l'élément hydratable est constitué d'un empilement d'au moins deux feuilles (20) d'hydrogel destiné à absorber le médicament, la structure hydratable comprenant un moyen (28) pour maintenir les feuilles adjacentes au moins partiellement séparées, et un moyen (26) destiné à maintenir les feuilles ensemble dans l'empilement.

2. Bioélectrode selon la revendication 1, dans laquelle les feuilles d'hydrogel (20) sont constituées d'une composition comprenant un polymère et un sucre.

3. Bioélectrode selon la revendication 2, dans laquelle le polymère est l'oxyde de polyéthylène, le polyacrylamide, l'alcool polyvinylique, l'amidon modifié, la cellulose ou des copolymères de ces polymères.

4. Bioélectrode selon l'une des revendications 1 à 3, dans laquelle le moyen de séparation (28) est constitué de granules ou de fibres disposés entre chaque paire de feuilles d'hydrogel (20) adjacentes afin de permettre l'écoulement du médicament entre les feuilles.

5. Bioélectrode selon la revendication 4, dans laquelle le moyen de séparation (28) est constitué de granules sous la forme de cristaux de sucre ou de particules de cellulose.

6. Bioélectrode selon l'une des revendications 1 à 5, dans laquelle les feuilles d'hydrogel (20) sont maintenues ensemble par sertissage, compression et découpage des bords (26) opposés des feuilles.

7. Bioélectrode selon l'une des revendications 1 à 6 comprenant un plateau de confinement (18) ayant un réceptacle de médicament.

8. Bioélectrode selon l'une des revendications 1 à 7, dans laquelle le moyen (12) permettant de maintenir l'élément hydratable et l'électrode ensemble est un film adhésif.

9. Procédé de fabrication d'une bioélectrode destinée à l'administration ionophorétique d'un médicament, qui comprend :
(a) la formation d'une masse d'hydrogel en feuilles (20),
(b) le séchage des feuilles d'hydrogel,
(c) l'immersion des feuilles séchées dans une solution de gonflement afin que les feuilles absorbent la solution, gonflent et se dilatent latéralement,
(d) le séchage des feuilles d'hydrogel gonflées,
(e) la distribution de granules (28) ou de fibres sur les feuilles,
(f) la disposition des feuilles en un empilement tel qu'elles soient maintenues séparées au moins partiellement par les granules ou les fibres,
(g) l'application à l'empilement de feuilles d'une électrode (14) destinée à recevoir un courant électrique destiné à produire un champ électrique et amener le médicament ionisé à migrer depuis l'élément hydratable jusque dans la peau ou le tissu sur lequel la bioélectrode est placée, l'électrode étant montée sur le moyen de fixation à proximité de l'élément hydratable.

10. Procédé selon la revendication 9, dans lequel l'étape (a) comprend :
* la mise en place d'hydrogel sur la surface extérieure d'un rouleau (46),
* la rotation du rouleau comportant l'hydrogel sur la surface d'une platine réfrigérée (45, 44) de manière à refroidir la couche externe du gel lorsqu'il entre en contact avec la platine et provoquer la congélation superficielle de celle-ci, son adhésion sur le plateau, et
* la séparation du plateau et de la masse d'hydrogel restant sur le rouleau pour former une feuille d'hydrogel.

11. Procédé selon la revendication 9 ou la revendication 10, dans lequel l'étape (b) comprend le séchage des feuilles d'hydrogel dans un four à convection à une température comprise entre 45 et 65°C.

12. Procédé selon l'une des revendications 9 à 11, dans lequel l'étape (c) comprend :
* la mise en place de feuilles d'hydrogel sur un écran,
* l'immersion de l'écran avec les feuilles d'hydrogel dans une solution d'eau et d'agent de raidissement afin d'amener les feuilles à gonfler et en finalement à se raidir.

13. Procédé selon l'une des revendications 9 à 12, dans lequel l'étape (e) comprend :
* le transport des feuilles d'hydrogel sous un mécanisme de distribution de granules ou de fibres afin de déposer les granules ou les fibres et faciliter leur fixation sur les feuilles, et
* l'application d'un brouillard ou d'une vapeur d'eau sur les feuilles et les granules ou les fibres pour humidifier les granules ou les fibres et faciliter leur fixation sur les feuilles.
